# EUROPEAN PATENT APPLICATION

(11) **EP 1 357 480 A1**
(43) Date of publication of application: **29.10.2003**
(21) Application number: 02100384.3
(22) Date of filing: 17.04.2002
(51) Int. Cl.: G06F 17/00, A61B 6/00, A61B 8/08, A61B 5/103, G01N 23/02

(54) **Osteoporosis screening method**

(71) Applicant: AGFA-GEVAERT, 2640 Mortsel (BE)
(72) Inventor: Dewaele, Piet, AGFA-GEVAERT, 2640, Mortsel (BE); Dispersyn, Gerrit,AGFA-GEVAERT, 2640, Mortsel (BE)

(57) **Abstract**

Results of a bone mineral density (BMD) measurement and a total score obtained by combining score values pertaining to the applicability of pre-defined risk factors are combined into a value indicating whether a person is at risk of osteoporosis.

## Description

### FIELD OF THE INVENTION

The present invention relates to a method of tracing of persons at risk of osteoporosis.

### BACKGROUND OF THE INVENTION

Osteoporosis is a widespread disease characterized by the loss of bone mass and deterioration of bone micro-architecture, resulting in the risk of bone fractures.
Of all parameters involved, low bone mass is the most important factor determining bone brittleness and fracture risk, particularly of the hip, spine and wrist.
Although osteoporosis affects more than 26 million persons in the United States alone, less than 10% of women with the disease and only 1% of men are diagnosed and receive treatment.
In order to prevent osteoporosis, or slow it down after it starts, it must first be known whether a person is at risk. The tracing of persons at risk in an asymptomatic population is termed screening.

A number of radiological screening tests have been proposed to detect low bone mass in asymptomatic persons.
One of the current non-invasive methods of bone mass measurement is bone mineral densitometry (BMD). Techniques to estimate BMD include conventional skeletal radiographs (Radiogrammetry and Absorptiometry), SPA (Single Photon Absorptiometry), DPA (Dual Photon Absorptiometry), DXA (dual-energy X-ray absorptiometry), quantitative computed tomography (QCT), and ultrasound (US).
The current gold standard for estimating BMD is DXA, which has gradually supplanted the oldest film-based techniques.
However, the DXA technique requires a large acquisition time hence a substantial time-to-diagnosis, well-trained operators and expensive equipment and hence its availability as a mass screening tool is limited. Although QCT is highly accurate in examining the anatomy and the density of transverse slices and trabecular regions in the spine, it is less practical as a routine screening test due to cost and high radiation exposure. DXA and QCT are axial techniques in that they provide measures of the spine and hip.

Although conventional skeletal X-rays recorded on film are used to detect bone disorders and fractures, they were of limited value in estimating bone mass.
However, with the advent of digital X-ray modalities such as computed radiography and direct (digital) radiography, the conventional bone mass estimation techniques have gained renewed interest because their inherent precision is substantially enhanced by digital measures.
The reported precision of radiographic absorptiometry and digital X-ray radiogrammetry is in the order of 1%, which is well in the range or below that of the established DXA.
Furthermore, these techniques are termed peripheral in that they analyze the appendicular skeleton, and hence are much quicker to operate in clinical practice.
However, in view of the fact that they provide a peripheral measure, the correlation of BMD with hip BMD and hip fracture risk is limited and questionable.

There are important limitations to screening as a means of preventing fractures. In a single measure of bone density, there is a small risk of inaccurate values, and there is no value of BMD that discriminates well between patients who develop a fracture and those who do not.

It is an object of the present invention to provide a screening method for identifying persons at risk of osteoporosis with enhanced success rate.

### SUMMARY OF THE INVENTION

The above-mentioned objects are realised by a method having the specific features set out in claim 1.

Specific embodiments of the present invention are set out in the dependent claims.

A specific aspect of this invention relates to a computer program product adapted to carry out the method of this invention when run on a computer.
The invention further relates to a computer readable carrier medium such as a CD-ROM comprising computer executable program code adapted to carry out the steps of the method of the present invention.

Still another aspect relates to a system for carrying out the method of the present invention. Such a system comprises a computer programmed to carry out the steps of the present invention upon input of BMD measurement results and values of risk factors collected e.g. by a questionnaire or through routine physical examination.

### Detailed description

Specific embodiments of methods for performing a bone mineral density measurement are radiogrammetry and radiographic absorptiometry. These techniques are simple and inexpensive, both of which features are important in times of economic constraints.

Radiogrammetry-based BMD estimation consists of performing geometric measurements in the plane of the image, and relating expressions of these measurements to BMD estimates and fracture risk. The basic assumption in radiogrammetry is based on the fact that, when osteopenia develops, the cortical thickness of the small tubular bones decreases, while the medullar cavity enlarges due to endosteal resorption. One measures the outside diameter D of the tubular bone, and it's inside diameter d.

The following are examples of radiogrammetric measures
- The cortical index = (D-d)/D, the combined cortical thickness = D-d. When endosteal resorption occurs, the cortical index and the combined cortical thickness decrease.
- Relative cortical area = 100*(D²- d²)/ D² and the cortical area = (D²- d²)
- Volume per area VPA = f*d*(1-d/D), in which f is a geometrical factor depending on the geometrical model assumed for the tubular bone (f=PI for a cylinder).

Methods of estimating BMD of a bone using digital X-ray radiogrammetry according to the principles outlined above are known in the prior art. For example in EP 1 046 374 A1 such a method has been disclosed.

Clinical studies report correlation of digital radiogrammetric VPA measure on the three middle metacarpals of 0.60 with DXA spine, 0.56 with DXA hip and 0.83 with DXA radius, the latter value plausibly being higher because of a corresponding skeletal site.

BMD measures based on radiographic absorptiometry (RA) compare the density in bony areas on film or on the digital image with the density of a simultaneous radiographed aluminum reference wedge. Usually these areas are the middle phalanxes of the three middle fingers. The result is expressed in equivalent aluminum thickness units. The software may encompass a soft tissue correction.

US5712892 discloses a method to estimate BMD on the basis of exposing a body extremity region and a calibration wedge with a continuous spectrum X-ray beam, an X-ray image converter for converting the X-ray image in a digital image signal, and an image processor to extract and convert the image signal in the region corresponding to the body extremity into a bone density measure.

US 6246745 and US 5917877 disclose methods to compute radiographic absorptiometry (of the hand wrist or calcaneous) based BMD measures using more exposures acquired by either multiple X-ray energies or by energy-selective multiple film.

Clinical studies report correlation of digital RA measures calculated on the middle phalanxes of digit II,III and IV of 0.66 with DXA spine, 0.55 with DXA hip and 0.76 with DXA radius, the latter value plausibly being higher because of a corresponding skeletal site. The difference between radiogrammetric and absorptiometric correlation with DXA is insignificant for DXA spine (p=0.30) and DXA hip (p=0.95), but is significant for DXA radius (p=0.07).

Another important method used for studying the peripheral skeleton is the quantitative ultrasound scanning (QUS) of the calcaneus (heel). Like the hand and wrist, the site is easily accessible, and the calcaneus has a high percentage of trabecular bone. Ultrasonic devices are attractive because they do not use ionizing radiation. Broadband ultrasonic attenuation (BUA) and speed of sound (SOS) are the main ultrasound variables measured, and they relate to both bone density and bone architecture (structure).
A clinical measure, called stiffness index, is provided based on these variables, and it indicates risk of osteoporotic fracture comparable to BMD as measured by DXA of the hip and spine.

Still other embodiments of a BMD measurement method are based on peripheral quantitive computer tomography (p-QCT) or on dual energy X-ray absorptiometry (p-DEXA).

Although any bone density measurement can be used to predict fracture risk in an individual, for site-specific prediction, the site of interest should be measured (i.e. measure the hip when predicting hip fracture risk).
Unfortunately, there is discordance between different BMD measurements performed at different sites in the same person, particularly in the early postmenopausal period.

As such, in practice, the choice of measuring the central or peripheral skeleton depends on age.
However, for the task of screening, an easily accessible skeletal site, such as the forearm, or heel is needed, and BMD measures at these sites only correlate moderately with axial BMD measures.

The inventors have found that additional factors, other than peripheral BMD measures, which are predictive of fracture risk, need be incorporated in the overall screening procedure to complement the deficiency associated with the use of peripheral BMD measures only. According to the invention score values pertaining to the applicability of pre-defined risk factors are entered in a computer. The values of the risk factors may e.g. be collected by having persons that are screened fill out a questionnaire or by routine physical examination.

Fracture risk may also be modelled by a combination of factors other than bone mineral density measures. This technique falls in the class of prognostic risk score (RS) models. Prognostic scoring models, or "risk score" models have explicit explanatory power and are simple to use. In such models, score values for selected independent variables are derived from normalised beta coefficients of logistic regression (or Cox proportional hazards regression) models as determined by the maximum c-index (equivalent to the area under the ROC curve). The score values are additive to a sum risk which is then correlated with historical risk.

Pre-defined risk factors are for example age, weight, length, body mass index, race, certain medication intake such as hormone replacement therapy and calcium intake, personal history of previous fractures and family history of osteoporosis, lifestyle factors such as smoking, alcohol use or physical activity.

Risk factors may include categorical and continuous variables. Continuous variables are for example age, weight, and length, body mass index. Attributes of bone geometry, such as tallness, hip axis length and femur length are continuous variables correlated with increased risk of fracture with a fall. Categorical variables are variables that assume only a discrete number of values and include for example ethnic type, smoking status, and physical activities.

From the evaluation of the values of these risk factors several score values may be deduced such as
- The Simple Calculated Osteoporosis Risk Estimation (SCORE). From a large pool of candidate factors, a reduced set was derived using a multivariate linear regression to model actual t-scores and a multivariate logistic regression to model risk of low bone density. The screening characteristics of the candidate linear and logistic regression models were evaluated by calculating the sensitivity and the specificity for the cut-point value of probability that gave 90% sensitivity.
   In its final form it uses terms each consisting of the following factors and associated weights for women:
   race (score 5) if not black
   + rheumatoid arthritis (score 4) if present
   + history of fracture (each fracture type of either hip, wrist or rib adds a score of 4)
   + (3*age)/10 (truncated)
   + hormone replacement therapy (score 1)
   - (weight in pounds/10) (truncated).
   A score ≥ 6 indicates a need for testing.
- The Osteoporosis Risk Assessment Instrument (ORAI) uses the following system: 15 points if age 75 or older, 9 points if 65 to 74 years, 5 points if 55 to 64 years, 9 points if weight < 60kg, 3 points if 60 to 69.9 kg and 2 points if not currently taking oestrogen. A score ≥ 9 indicates the need for testing.
- The Age, Body Size, No Oestrogen score (ABONE) uses the following scoring system: one point if age > 65 years, one point if weight < 63.5kg; and one point if never taken an oral contraceptive or oestrogen for at least six months. A score ≥ 6 indicates a need for testing. The body weight criterion simply uses a weight < 70kg as the criterion for testing.

Despite their simplicity, the performance of risk assessment methods exclusively based on demographic and patient data is considered to be too low to be used as the sole basis for establishing a mass-screening program.

The inventors of the present invention have found that combinations of the BMD measure and the above risk factors result in increased performance.

The way in which the individual score values pertaining to pre-defined risk factors are combined may broadly be classified into two groups according to the level of combination (1) either the features are combined in a single classifier structure or (2) classifications of the features are combined by a second stage classifier.

### (1) Combined feature classification

In the screening procedure according to the present invention where *d* measures of osteoporosis risk are collected, a mapping from **R**^{*d*} into the set {at risk, not at risk} is to be realised.
Such a mapping characterises a classifier having a *d*-dimensional input vector and two output classes.

Several combined feature classifiers are applicable.

Parametric classifiers assume that the feature of each class obeys a class-conditional feature probability density function (PDF), characterized by a few number of parameters.

A mean value vector and a feature covariance matrix e.g. characterize a multidimensional normal distribution (multivariate Gaussian, MVG).
Learning from the data is reduced to estimating this fixed number of model parameters. If the hypothesized model is different from the actual one, this form of classifier is bound to suffer from model-mismatch errors.
An example of such a classifier is a linear or quadratic Bayesian classifier.

Non-parametric classifiers do not impose restrictions on the underlying feature probability density functions. Learning from data involves a more elaborate step of estimating a much larger number of parameters (e.g. a Gaussian mixture model, GMM). This type of classifier may suffer from poor generalization when the data are overfit, resulting in a mismatch between training and test data sets.

The K-nearest neighbor (K-NN) classifier falls in this category. Specialisations of this type are nearest neighbor (NN) classifiers, weighted K-NN classifiers, k-means clustering and vector quantization.

Boundary-decision classifiers attempt to learn from data linear or nonlinear boundary functions that separate classes. Multi-layer perceptrons, linear and polynomial decision tree classifiers and artificial neural networks fall into this category. For example, a linear decision tree defines a polyhedral subdivision of space; it is a classifier if no leaf region contains points from other classes. Classifiers of this generally require long training time, but can be effective if classes are separable by a well-defined boundary function.
Other examples of this paradigm are support vector machines (SVM) and kernel methods.

### (2) Combination of classification decisions

There exist many possibilities to combine classifier decisions, the general combination schemes having wider applicability than the special combination rules.
This area of pattern recognition is termed information fusion, with information being data, features or decisions.
Data fusion is concerned with the problem of how to combine data from multiple sensors to perform inferences that may not be possible from a single sensor alone.
Data fusion can combine several unreliable data measurements in a feature to produce a more accurate signal by enhancing the common signal and reducing the uncorrelated noise.
When pertaining to decisions, fusion is divided into two broad categories: (a) heuristic approaches, and (b) methods based on probability theory and statistics.

Heuristic approaches try to mimic human ways in making decisions. Voting strategies fall in this class. Each feature is classified by an expert rule, and gets one vote. The classification problem is then reduced to a *k*-out-of-*n* voting technique. When at least *k*-out-of-*d* numbers of risk factors are present, the result of this technique will be that the patient is referred for clinical follow-up and more extensive axial BMD measurements, such as DXA.
For some values of k, particular decision fusion schemes are obtained:
- *k*=1. This amounts to OR-ing the individual binary classification of each risk factor. Hence when at least one risk factor is present, the patient is considered to be at risk. In a screening setting, this means that more patients will be considered for referral, hence the TPR will be higher, and few patients who are actually at risk will be missed, but the number of patients referred will also be higher. Specificity (TNR) will be lower.
- *k=d*. This is the AND rule. The person is only referred when all *d* risk factors are present. This will prevent over-referral in that the number of patients that test positively are lower due to more severe risk conditions imposed. The TPR will be smaller since the number of FN increases and the number of TP decreases by the same amount.
- *K=(d+1)*/*2*. This is the MAJORITY rule. It is a compromise between the AND and the OR rule.

Other examples of applicable combinations of the decisions based on the results of the BMD measurement and the total score of the risk factors are e.g. a logical operator based on fuzzy logic using multiple-valued classification of individual features or a mathematical technique based on Bayesian inference using probabilities or based on the Dempster-Shafer theory of evidence.

### Measuring classifier performance

A classifier's performance can be tested by estimating its misclassification rate. However, for the task of medical screening, it makes more sense to optimise alternative objectives.

A typical approach is to analyse the screening performance by means of a confusion matrix (Table 1). One axis of this matrix depicts the classification values of the test, which is a binary variable in the case of screening (either positive or negative screening test outcome), the other axis depicts the truth value (the person actually has the disease, or runs the risk of getting osteoporosis).

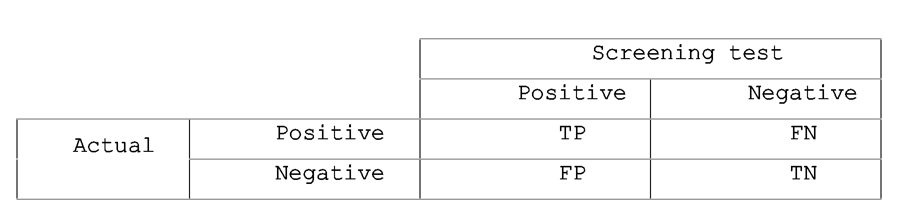

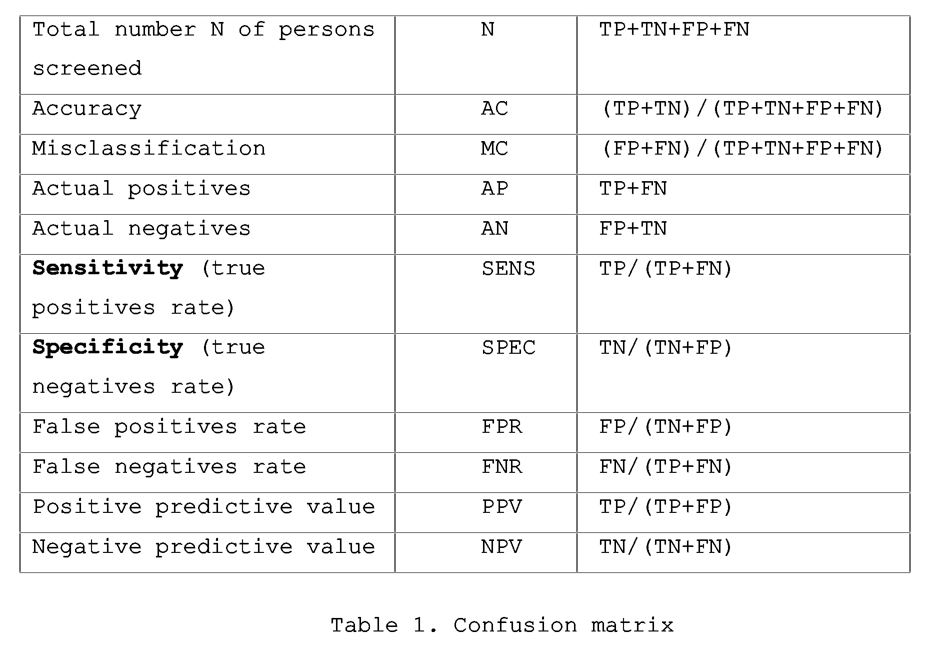

Instead of minimising the misclassification rate, it is preferable to maximize the sensitivity or true positives rate (SENS) and specificity or true negatives rate (SPEC) of a diagnostic or screening model.
The "sensitivity" denotes the probability that a test result will be positive when the disease is present.
The "specificity" denotes the probability that a test result will be negative when the disease is absent.
The "positive predictive value" denotes the probability that the disease is present when the test is positive.
The "negative predictive value" denotes the probability that the disease is absent when the test is negative.

There are several reasons why such objectives are more appropriate. For example,
- the misclassification costs for false positives (FP) or false negatives (FN) may be different. The cost of falsely referring a positively screened person to a more expensive BMD test is generally different of the cost of not referring a negatively screened person who actually should receive appropriate follow-up or treatment in order to prevent more intervening and costly measures due to non-traumatic fractures in a later stage. These socio-economic costs should be balanced against one another in the setting of mass population screening.
- The real class distributions may be highly skewed, with the positive cases composing just a small fraction of the population, and thus the misclassification rate (MC) or the accuracy (AC) may provide a highly biased measure of model performance. Indeed, from the definition of MC=(number of FP + number of FN)/(total number of screened persons), MC and AC could be highly biased by one or either type of errors. Due to the proportional weighting in the definition of the MC, the MC will always be closer to that type of error (FPR of FNR), which has been obtained by the largest number of persons in the respective groups.

Therefore, the simultaneous reduction of false negative and false positive classifications, which is critical in evaluating the screening performance of a procedure or test, is often dealt with the receiver operating characteristic (ROC) analysis. ROC analysis specifies the performance of a classifier in terms of sensitivity and specificity, by plotting the true positive rate (sensitivity) versus the false positive rate, which is 1-specificity, as a function of a cut-off threshold employed to assign the test data to either positive or negative cases. The area under the ROC curve (AUC) indicates the discriminating power of the test. An area of 0.8 e.g. means that a randomly selected individual from the actual positive group has a test value larger than that of a randomly chosen individual from the negative group in 80% of the cases.
The ROC curve can be used to indicate or derive the optimal cut-off threshold for the test, for example the cut-off threshold corresponding to the upper-leftmost point on the curve, that is the point, which is closest to the ideal point of simultaneous maximal sensitivity (100%) and specificity (100%).

The chosen point is called the working point of the screening test. The chosen point in a screening setting may be based on economical factors in that the maximum allowed false negatives (the missed cases) is imposed.

When the AUC is 0.5, the test under study cannot distinguish between the two groups. AUC equal to 1.0 means that there is a perfect separation of the two groups.

Clinical studies, that measure the screening power of single peripheral BMD measures with respect to DXA as a gold standard, (that is: a person is considered at risk when DXA detects axial osteopenia) yield a sensitivity and specificity at the highest negative predictive value (the probability that the person does not run the risk when the test is negative) working point of
- 80.4% and 52.3% resp. for a radiogrammetric measure based on VPA of 3 metacarpals, at FN percentage of 11%.
- 84.3% and 51.8% resp. for a absorptiometric measure of 3 phalanxes, at a FN percentage of 9.8%

However, when these BMD measures are combined with a score value based on demographic and clinical data (OR type of fusion), these values of sensitivity and specificity increase to (at the highest negative predictive value working point)
- 90.0% for the radiogrammetric measure, at a FN percentage of 4.6%
- 91.8% for the absorptiometric measure, at a FN percentage of 3.8%.

Hence the sensitivity of either of the single measures is substantially increased and the FN percentage (the fraction of patients not detected to be at risk (test negative) when they actually are) is substantially reduced by the present invention by suitably combining them with a patient questionnaire score.

Lifetime fracture risk (LFR) and the remaining lifetime fracture risk (RLFR), expressing the probability that a person will incur a fracture in one year or a decade traditionally based on the person's age and an axial BMD value, may be computed and refined on the basis of BMD data supplemented by more extensive demographic and patient data and scores.

## Claims

1. A method of tracing persons at risk of osteoporosis comprising the steps of
- performing a bone mineral density (BMD) measurement on said person thereby generating a BMD measure,
- obtaining score values pertaining to the applicability of pre-defined risk factors to said person,
- calculating a total score by means of said score values,
- combining said BMD measure and said total score into a value indicating whether said person is at risk of osteoporosis.

2. A method according to claim 1 wherein said total score is obtained by weighting each of said score values and totalling all weighted score values.

3. A method according to claim 1 wherein said value indicating whether a person is at risk of osteoporosis is the result of a classifier operating on said total score and said BMD measure.

4. A method according to claim 3 wherein said classifier is one of
- a parametric classifier such as a linear or quadratic Bayesian classifier,
- a non-parametric classifier such as a K-nearest neighbour classifier (KNN), a binary decision tree (BDT) or an Artificial Neural Network (ANN) or support vector machines (SVM).

5. A method according to claim 1 wherein said value indicating whether said person is at risk of osteoporosis is the result of a combination of a decision based on said BMD measure and a decision based on said total score.

6. A method according to claim 5 wherein said combination is one of
- a logical operator based on Boolean algebra such as OR and AND,
- a logical operator based on fuzzy logic using multiple valued classifications of individual features,
- a mathematical technique based on Bayesian inference using probabilities or based on the Dempster-Shafer Theory of Evidence.

7. A computer program product adapted to carry out the steps of any of claims 1 to 6 when run on a computer.

8. A computer readable carrier medium comprising computer executable program code adapted to carry out the steps of any of claims 1 to 6.

9. A system for tracing persons at risk of osteoporosis comprising a computer programmed to carry out the steps of any of claims 1 to 6 upon input of BMD measurement results and values of risk factors.
